# EUROPÄISCHE PATENTANMELDUNG

(11) **EP 0 945 116 A2**
(43) Veröffentlichungstag der Anmeldung: **29.09.1999**
(21) Anmeldenummer: 99106092.2
(22) Anmeldetag: 26.03.1999
(51) Int. Cl.: A61H 31/00

(54) **Vorrichtung zur Unterstützung des Blutkreislaufes**

(30) Priorität: 27.03.1998 DE 19813836
(71) Anmelder: Deiwald, Johannes, 79241 Ihringen (DE)
(72) Erfinder: Deiwald, Johannes, 79241 Ihringen (DE)
(74) Vertreter: COHAUSZ HANNIG DAWIDOWICZ & PARTNER

(57) **Zusammenfassung**

Die Erfindung betrifft eine Vorrichtung zur Unterstützung des Körperblutkreislaufes und zur Entlastung des Herzens mittels auf den Körper eines Patienten ausübbaren Unterdrucks mit einer Kammer in der ein zu behandelnder Patient einliegt und mit einer Atemvorrichtung zur Atemluftversorgung des Patienten wobei die Kammer mit Flüssigkeit füllbar ist und in ihr herzschlagsynchrone, auf den Körper des Patienten einwirkende Unterdruckimpulse erzeugbar sind.

Die Erfindung betrifft weiterhin eine Vorrichtung zur Unterstützung des Lungenblutkreislaufes und zur Entlastung des Herzens mit einer Atemmaske, über die der Patient mit Atemluft versorgt wird, bei der im durch Atemmaske und Patient gebildeten Atemvolumen herzschlagsynchrone Unterdruckimpulse erzeugbar sind.

## Beschreibung

Die Erfindung betrifft eine Vorrichtung zur Unterstützung des Körperblutkreislaufes und zur Entlastung des Herzens mittels auf den Körper eines Patienten ausübbaren Unterdrucks mit einer Kammer in der ein zu behandelnder Patient einliegt und mit einer Atemvorrichtung zur Atemluftversorgung des Patienten.

Die Erfindung betrifft weiterhin eine Vorrichtung zur Unterstützung des Lungenblutkreislaufes und zur Entlastung des Herzens mit einer Atemmaske, über die der Patient mit Atemluft versorgt wird, sowie eine Kombination aus beiden Vorrichtungen zur allgemeinen Kreislaufunterstützung.

Bereits aus der DE 4 137 154 ist es bekannt, Patienten mit Unterdruck zu behandeln. Das genannte Dokument offenbart eine Differenz-Unterdruckkammer, in der sich ein zu behandelnder Patient befindet, der über eine Atemvorrichtung mit Atemluft versorgt wird.

Mittels einer handelsüblichen Vakuumpumpe wird aus der Kammer Luft abgesaugt, so daß der Körper des Patienten einem permanenten Unterdruck ausgesetzt ist.

Durch das Absaugen der Luft ergibt sich zwischen der Kammerluft und der Atemluft ein Differenzdruck, so daß aufgrund des größeren Atemluftdruckes die Lunge des Patienten von innen aufgebläht wird.

Eine derartige permanente Unterdruck-Behandlung bewirkt eine Ausdehnung des Körpers und insbesondere der Adern und Venen. Darüber hinaus erzeugt der Körper mehr rote Blutkörperchen, wodurch eine Sauerstoffanreicherung ermöglicht wird. Diese Vorrichtung dient also im wesentlichen einer Sauerstoffsättigungstherapie.

Demgegenüber hat die Behandlung jedoch den Nachteil, daß aufgrund des Differenzdruckes und insbesondere des erhöhten Atemluftdruckes ein permanenter Druck über die Lunge auf das Herz ausgeübt wird. Eine derartige Differenzunterdruck-Behandlung ist dementsprechend durch eine erhöhte Herzbelastung gekennzeichnet.

Aufgabe der Erfindung ist es eine Vorrichtung bereitzustellen, mit der neben einer Unterstützung des Blutkreislaufes auch eine Entlastung des Herzens erreicht werden kann.

Diese Aufgabe wird zur Unterstützung des KörperBlutkreislaufes dadurch gelöst, daß die eingangs genannte Kammer, in der der zu behandelnde Patient einliegt, mit Flüssigkeit füllbar ist und in der Kammer impulsartig und herzschlagsynchron Unterdruck erzeugbar ist, der insbesondere kurzzeitig auf den Körper des Patienten einwirkt.

Gegenüber dem Stand der Technik zeichnet sich die Kammer also dadurch aus, daß statt-der permanenten Einwirkung eines Unterdrucks auf den Körper des Patienten hier nur mit einer kurzzeitigen impulsartigen und herzschlagsynchronen Einwirkung des Unterdrucks auf den Körper des Patienten, d.h. mit Unterdruckimpulsen gearbeitet wird.

Um die impulsartige und zeitlich vorherbestimmbare Einwirkung des Unterdrucks auf den Körper zu ermöglichen, ist die Kammer anstatt mit Luft oder Gas mit einer Flüssigkeit gefüllt, die sich gegenüber der Luft durch ihre Inkompressibilität auszeichnet, so daß ein in der Kammer impulsartig erzeugter Unterdruck instantan und exakt nur über die Dauer seiner Erzeugung auf den Körper ausgeübt wird. Die Dauer bewegt sich hierbei typischerweise zwischen 0.05 und 0.2 Sekunden. Je nach Anwendungsfall sind aber auch hiervon abweichende Werte möglich.

Bei einer Verwendung von Luft anstatt von Flüssigkeit, wie z.B. Wasser, würde es hingegen lediglich zu Dichteschwankungen innerhalb der Kammer kommen, die sich mit Schallgeschwindigkeit innerhalb der Kammer und damit unkontrolliert ausbreiten.

Im Rahmen einer Behandlung wird der Körper des Patienten während der Herzkammererregung, d.h. am Anfang des systolischen Blutdruckaufbaus einem kurzen, impulsartigen Unterdruck von z.B. 0.1 Sek. ausgesetzt, wobei dieser Unterdruckimpuls herzschlagsynchron bzw. herzschlagkorreliert erzeugt wird.

Der impulsartige Unterdruck verursacht eine Ausdehnung der Blutgefäße und des Kapillarbettes im Körper, was zu einem relativen Unterdruck in den Blutgefäßen führt. Dementsprechend -pumpt das Herz das Blut mit geringerem Widerstand in die Blutgefäße und muß weniger Arbeit leisten. Hierdurch sinkt der Druck auf die Innenschichten des Herzens und der Blutgefäße, so daß diese Innenschichten entlastet und gut versorgt werden können.

Während eines Herzschlages dehnen sich die Aorta und andere größere Arterien unter dem Druck des gepumpten Blutes aus, wobei durch die anschließende Kontraktion das Blut weitergepumpt wird. Ausdehnung und Kontraktion dieser Arterien wird durch die auf den Körper einwirkenden herzschlagsynchronen Unterdruckimpulse unterstützt und somit das Herz entlastet, so daß die gesamte Vorrichtung eine Art von unterstützender Herztätigkeit übernimmt.

Bei dem durch die Vorrichtung hervorgerufenem Pumpeffekt, wird durch die Herz- und Venenklappen dafür gesorgt, daß das gepumpte Blut immer nur in eine Richtung fließt.

Während der Blutdruck von dem systolischen Blutdruck auf den diastolischen Blutdruck sinkt, wird auch der impulsartig und unterstützend wirkende Unterdruck anhand von voreingestellten Daten durch ein an der Vorrichtung vorgesehenes Steuergerät abgesenkt.

Hierdurch wird eine Blutgefäßverengung verursacht, so daß das Gefäßvolumen insgesamt kleiner wird. Das Blut wird somit in die Vorhöfe des Herzens gefördert, so daß die Herzkammern optimal mit Blut gefüllt werden. Dies steigert den systolischen Blutausstoß, was zu einer besseren Durchblutung pro Herzschlag und einer Herzfrequenzabsenkung führt (vagale Stimmulation).

Die erfindungsgemäße Vorrichtung ermöglicht somit eine Entlastung, längere Ruhepause und bessere Durchblutung des Herzens. Weiterhin werden unabhängig vom Herz- und Blutgefäßzustand die Durchblutung und systolischer bzw. diastolischer Blutdruck stabilisierbar.

Durch die mittels der Vorrichtung optimal stabilisierte Durchblutung wird der Transport von Sauerstoff und Nährstoffsubstanzen zu den Zellen hin und ebenfalls der Abtransport von Stoffwechselendprodukten von den Zellen weg gesteigert.

Während des systolischen Blutdruckaufbaus durch das Herz und dem herzschlagsynchronen Unterdruckaufbau in der Kammer durch die Vorrichtung erfolgt somit eine Unterstützung der Diffusion von Nährstoffen, Hormonen und Sauerstoff aus dem Blut in die Zellen, d.h. von Gebieten steigenden Blutdruckes zu den Gewebszellen in denen ein niedriger Blutdruck vorherrscht, bis daß sich diese Druckdifferenzen ausgeglichen haben.

Im umgekehrten Fall wird bei sinkendem Blutdruck und gezieltem abnehmenden herzschlagsynchronen Unterdruckimpuls die Diffusion von Ausscheidungsstoffen aus den Zellen in das Blut, d.h. wiederum von den Gebieten höheren Zellendruckes in die Gebiete niedrigeren sinkenden Blutdruckes, die in den Kapillaren vorliegen, begünstigt.

Der gesamte Stoffwechsel wird somit optimiert, biochemische Lebensvorgänge verlaufen zügiger, ohne Komplikationen und Rückstände, was für Erholung, Regeneration und Heilung die Voraussetzung ist. Weiterhin funktionieren sämtliche Organe optimal, das Herz wird entlastet, auf eine geringere Frequenz umgestellt und gut versorgt. Zusätzlich wird das Immunsystem durch eine Unterstützung der Lymphtätigkeit gekräftigt und die Anpassungsfähigkeit des Körpers somit erhöht.

Um in der Kammer impulsartig und herzschlagsynchron einen Unterdruck zu erzeugen, wird bevorzugt an der Kammer ein Unterdruck-Erzeuger angeordnet, mit dem impulsartig das Kammervolumen änderbar ist. Zur Änderung des Kammervolumens weist dieser Unterdruck-Erzeuger vorteilhafterweise eine elastisch verformbare Membran auf, die insbesondere von einen elektromagnetischen Antrieb bewegbar ist.

Durch eine steuerbare Auslenkung der verformbaren Membran kann somit das Volumen in der Kammer geändert werden. Da sich jedoch aufgrund der Inkompressibilität der Flüssigkeit das Flüssigkeitsvolumen nicht ändert, geht mit der Änderung des Kammervolumens eine Änderung des Körpervolumens des Patienten einher. Da das Volumen in der Kammer und auch in der später beschriebenen Atemmaske sowohl vergrößert als auch verkleinert werden kann, kann auf den Körper des Patienten auch gezielt ein impulsartiger Überdruck ausgeübt werden, sofern dies gewünscht sein sollte.

Dementsprechend dehnt sich bei einer Unterdruckerzeugung in der Kammer der Körper des Patienten aus, was eine Blutgefäßerweiterung mit den eingangs genannten Wirkungen hervorruft.

Der elektromagnetische Antrieb des Unterdruckerzeugers wird bevorzugt in einfacher Weise durch einen Permanentmagneten gebildet, der sich in dem Magnetfeld einer elektrisch ansteuerbaren Spule befindet.

Ebenfalls ist ein umgekehrter Aufbau möglich, wie er im wesentlichen einem Lautsprecheraufbau entspricht. Hierbei würde die elastisch verformbare Membran des Unterdruckerzeugers der Membran des Lautsprechers entsprechen.

Daneben ist es ebenfalls denkbar, den Antrieb mit Hilfe anderer üblicherweise-einsetzbarer elektromagnetischer oder elektromechanischer Vorrichtungen zu realisieren. Wichtig ist hierbei lediglich, daß der Antrieb des Unterdruckerzeugers durch ein Steuergerät ansteuerbar ist.

Als Antrieb für die Unterdruckerzeuger kommen Vorrichtungen in Betracht, in denen der elektrische Ansteuerimpuls in eine mechanische Aktivität umgewandelt werden kann. Dementsprechend können elektromagnetische, elektrodynamische oder elektrostatische sowie auch Kristallwandler zum Einsatz kommen. Einzige Voraussetzung ist, daß diese Antriebe bei Infraschallfrequenzen, d.h. unterhalb von 16 Hertz arbeiten können, um herzschlagsynchron einsetzbar zu sein.

Um die Ansteuerung des elektromagnetischen Antriebs des Unterdruckerzeugers zu realisieren, umfaßt die erfindungsgemäße Vorrichtung, d.h. hier die flüssigkeitsgefüllte Kammer, Geräte zur Überwachung der Herz- und/oder Blutkreislauftätigkeit. Mittels dieser Geräte sind Herzströme (Aktionsströme, QRS), Herztöne oder Ultraschallbilder des Herzens, die Herztätigkeit, der Blutdruck oder der Blutstrom überwachbar.

Mittels eines an diesen Geräten angeschlossenen Steuergerätes, wie z.B. einem Computer, sind sodann aus den gewonnenen Informationen Impulse zur Ansteuerung des Unterdruckerzeugers ableitbar. Die Impulse werden berechnet aus den Überwachungsdaten, sowie weiterer Vorrichtungsparameter und vom Arzt eingegebener Daten des Patienten/der Vorrichtung. Insofern ist gewährleistet, daß synchron oder korreliert mit dem Herzschlag des Patienten Impulse zur Unterdruckerzeugung in der Kammer bereitgestellt werden.

Hierbei kann beispielsweise mit jedem Herzschlag ein Unterdruckimpuls in der Kammer oder auch nur mit ausgewählten Herzschlägen, d.h. z.B. mit jedem zweiten oder dritten Herzschlag ein Unterdruck in der Kammer hervorgerufen werden. Dies gilt nicht nur für die hier diskutierte Kammer, sondern auch für die später beschriebene Atemmaske.

Weiterhin ist es besonders vorteilhaft, wenn die Impulse zur Ansteuerung des Unterdruckerzeugers in ihrer Form und/oder Höhe einstellbar sind. Hierdurch ergibt sich eine gezielte Einstellbarkeit der Größe des gewählten Unterdruckes sowie der Verlaufskurven des ansteigenden oder abfallenden Unterdruckes. Dementsprechend lassen sich die eingangs genannten Diffusionsmechanismen im Körper, d.h. die Aufnahme von Nährstoffen und die Abgabe von Stoffwechselprodukten gezielt beeinflussen.

Es kann daher mit den verschiedenen Formen und Stärken, der aus den elektrischen Impulsen abgeleiteten Unterdruckimpulsen, eine unterschiedliche Wirkung bei der Durchblutung, dem Blutdruck und dem Stoffwechsel erreicht werden.

Schon ein kleiner Druckunterschied bei den unterstützend ausgeübten Unterdruckimpulsen wirkt sich auf die Durchblutung stark aus. Eine zeitliche Verschiebung des maximalen systolischen Blutdruckes in der Blutdruckkurve mit Hilfe der geformten herzschlagsynchronen Unterdruckimpulsen wirkt sich dabei auf den Stoffwechsel aus. Durch eine derartige Verschiebung können die Versorgungs- und Entsorgungszeiten im Stoffwechsel ausgeglichen oder in eine gewünschte Richtung beeinflußt werden.

Weiterhin bewirkt die erfindungsgemäße Vorrichtung, daß unabhängig vom Zustand der Blutgefäße und der Herztätigkeit der systolische und diastolische Blutdruck von Form und Stärke der herzschlagsynchronen Unterdruckimpulse abhängen.

Besonders vorteilhaft ist es auch, wenn an der flüssigkleitgefüllten Kammer Drucksensoren vorgesehen sind, mit denen der Druck in der Kammer überwachbar ist. Insbesondere ist es vorteilhaft, wenn zwei verschiedene Drucksensoren angeordnet sind, die zur Überwachung eines unteren und eines oberen, in der Vorrichtung herrschenden Druckgrenzwertes dienen.

Aus den gemessenen Druckwerten und der überwachten Herztätigkeit, sowie eventuell von einem behandelnden Arzt vorgegebenen äußeren Parametern, sind mittels des erwähnten Steuergerätes elektrische Impulse ableitbar, mit denen der Unterdruckerzeuger angesteuert wird.

Um zu verhindern, daß der Druck in der Kammer bereits mit der Ein- und Ausatmung des Patienten, wobei sich der Körper entsprechend ausdehnt, verändert, ist es vorgesehen, daß mit der Kammer ein Kompensationsbehälter mit variablem Inhaltsvolumen verbunden ist.

Die bei der Einatmung, durch die Zunahme des Körpervolumens verdrängte Flüssigkeit kann somit in den Kompensationsbehälter entweichen, so daß der Druck in der Kammer während des Ein- und Ausatmenvorganges konstant bleibt.

Das variable Inhaltsvolumen des Kompensationsbehälters ergibt sich entweder dadurch, daß dieser Behälter gegen die Atmosphäre hin geöffnet ist oder aber durch eine flexible verformbare Membran abgeschlossen ist. Diese flexible Membran gibt sodann unter dem Einfluß des ein- bzw. ausströmenden Flüssigkeitsvolumens nach, so daß eine Veränderung des Druckes in der Kammer vermieden wird.

Die Anordnung einer Membran am Kompensationsbehälter hat darüber hinaus weiterhin den Vorteil, daß diese Membran mit einem weiteren Antrieb versehen werden kann, so daß es möglich ist, zusätzlich zu den impulsartig ausgeübten Unterdruckimpulsen eine Beinflussung des Druckes in der Kammer vorzunehmen, wodurch beispielsweise eine unterstützende Beatmung des Patienten realisiert werden kann.

So wird, durch eine entsprechende Auslenkung der Membran des Kompensationsbehälters, z.B. dessen Volumen vergrößert, so daß Flüssigkeit aus der Kammer in den Kompensationsbehälter einströmt und auf den Körper des Patienten einen kontinuierlich ansteigenden Unterdruck ausübt, so daß der Patient automatisch, aufgrund der Ausdehnung des Brustkorbes und der damit einhergehenden Vergrößerung seines Lungenvolumens einatmet.

Im umgekehrten Fall, kann ebenfalls die Ausatmung des Patienten unterstützt werden.

Um zu vermeiden, daß der bewußt impulsartig in der Kammer erzeugte Unterdruck ebenfalls durch ein Ausweichen der Flüssigkeit in den Kompensationsbehälter kompensiert wird, ist es bevorzugt vorgesehen, daß die Verbindung zwischen der Kammer und dem Kompensationsbehälter über ein ansteuerbares Ventil verschließbar ist. Dieses Ventil kann beispielsweise ebenfalls über das Steuergerät angesteuert werden, welches auch für die Steuerung des Unterdruckerzeugers eingesetzt wird.

Dementsprechend wird unmittelbar vor Ansteuerung des Unterdruckerzeugers das Ventil zwischen Kompensationsbehälter und Kammer geschlossen, so daß der während des Herzschlages wirkende impulsartige Unterdruck nicht verloren geht und auf den Körper des Patienten einwirken kann. Alternativ kann auch eine Widerstandsdüse in der Verbindung vorgesehen sein.

Die Dauer des auf den Patienten einwirkenden Unterdruckimpulses, sowie die entsprechende Verschlußzeit des genannten Ventiles beträgt etwa nur 0,1 sec. Nach dieser Zeit ist der Unterdruck in der Kammer wiederum abgebaut, so daß das Verbindungsventil erneut geöffnet werden kann, um eine Druckkompensation während der Atmung des Patienten in der Kammer zu gewährleisten.

Um dem Patienten in der Kammer einen angenehmen Aufenthalt zu ermöglichen, kann die Kammer beispielsweise ein Liegenetz aufweisen, auf dem der Patient zu liegen kommt.

Alternativ oder zusätzlich kann die Flüssigkeit in der Kammer ebenfalls eine derart hohe Dichte aufweisen, daß der Patient in der Flüssigkeit leicht aufschwimmt oder sogar schwebt. Dies kann beispielsweise in einfacher Ausführung derart geschehen, daß in der Flüssigkeit ein Salz gelöst wird, um den Auftrieb des Patienten in der Flüssigkeit, wie z.B. Wasser, zu erhöhen.

Ebenfalls ist es vorteilhaft, wenn an der Kammer zur Temperierung der Kammerflüssigkeit eine Heizvorrichtung vorgesehen ist. Insofern kann die Kammerflüssigkeit auf einer Temperatur gehalten werden, die der in der Kammer liegende Patient als angenehm empfindet.

Weiterhin ist es wichtig, daß an der Kammer ansteuerbare Zufluß-, Abfluß- und End- bzw. Belüftungsventile angeordnet sind. Dieses ermöglicht besonders in Notfall-Situationen, daß ein Abfluß- und Belüftungsventil automatisch geöffnet werden, um eine automatische Entleerung der Kammer, z.B. bei Stromausfall oder auf Wunsch des Patienten zu gewährleisten.

Die Kammer, die einen großen Deckel zum erleichterten Einstieg aufweist, wird üblicherweise etwas länger sein als die zu behandelnde Person und in der horizontalen Ausrichtung aufgestellt sein. Für den Fall, daß der Einfluß des hydrostatischen Druckes, d.h. eine Druckänderung in der Kammer von einem Ende zum anderen Ende bewußt eingesetzt werden soll, ist es ebenfalls möglich, die Kammer aus Ihrer horizontalen Position zu verschwenken. Diese Verschenkung kann bis in die vertikale Position erfolgen.

Die beschriebene Kammer kann ebenfalls derart ausgebildet sein, daß sich nur der Körper des Patienten, nicht aber der Kopf in der Kammer befindet. Hierzu kann die Kammer an einer Stelle eine entsprechende Schleuse aufweisen, die eine Dichtfunktion übernimmt und beispielsweise den Hals- oder Schulterbereich des Patienten umgibt.

Alternativ oder zusätzlich zur Körperblutkreislauf-Unterstützung und zur Entlastung des Herzens kann auch der Lungenblutkreislauf durch eine entsprechende Vorrichtung unterstützt werden. Diese Vorrichtung ist im wesentlichen durch eine Atemmaske gebildet, über die der Patient mit Atemluft versorgt wird. Hierzu braucht der Patient nicht in der zuvor beschriebenen Kammer liegen. Das durch die Atemmaske und das Lungenvolumen des Patienten gebildete Atemvolumen wird bei dieser Vorrichtung impulsartig und herzschlagsynchron geändert, so daß innerhalb des gegebenen Atemvolumens ein kurzzeitiger Unterdruck erzeugbar ist, der auf das Atmungssystem des Patienten einwirkt.

Um dies zu erreichen, ist genauso wie bei der Kammer, an der Atemmaske ein Unterdruckerzeuger angeordnet, mit dem das Atemvolumen änderbar ist. Dieser Unterdruckerzeuger kann m wesentlichen den gleichen Aufbau haben, wie der bei der Kammer beschriebene Unterdruckerzeuger, d.h. er umfaßt eine verformbare Membran, die insbesondere von einem elektromagnetischen Antrieb bewegbar ist.

Die Ansteuerung dieses elektromagnetischen Antriebes erfolgt in der gleichen Weise durch dasselbe oder ein anderes, wie auch schon bei der Kammer beschriebene Steuergerät. In der Atemmaske, durch die der zu behandelnde Patient mit Atemluft versorgt wird, sind in vorteilhafter Weise Ventile angeordnet, die die Strömungsrichtung der Atemluft bestimmen. Hierdurch wird im wesentlichen erreicht, daß eine Pendelatmung vermieden wird, um den Patienten immer mit sauerstoffreicher Luft zu versorgen.

Besonders vorteilhaft ist es, wenn die in der Atemmaske vorgesehenen Ventile ansteuerbar sind. Hierbei können sowohl die zur Bestimmung der Stömungsrichtung vorgesehenen Ventile ansteuerbar sein, als auch zusätzliche Ventile, die ergänzend einsetzbar sind.

Während der Herzkammererregung, die dem systolischen Blutdruckaufbau entspricht, werden durch Ansteuerung der Ventile, beispielsweise durch das eingangs genannte Steuergerät, die Ventile geschlossen und die Luftzufuhr abgesperrt. Alternativ können statt der ansteuerbaren Ventile auch Widerstandsdüsen vorgesehen sein. Im Anschluß daran, wird über den Unterdruckerzeuger, der ebenfalls von dem Steuergerät angesteuert wird, ein impulsartiger, herzschlagsynchroner Unterdruck im Atmungssystem des Patienten erzeugt.

Dies führt zu einem relativen Unterdruck in den Blutgefäßen, nämlich zum einem in den Gefäßen die für den Gasaustausch zuständig sind und unterstützt somit den gesamten Blutstrom des Lungenblutkreislaufes und zum anderen in den Gefäßen der Eigenversorgung und unterstützt hierdurch teilweise auch den Blutstrom des Körperblutkreislaufes.

Der Unterdruckimpuls, der herzschlagsynchron beispielsweise nur über eine Dauer von 0,1 Sec. erzeugt wird, wirkt durch das Zwerchfell ebenfalls auch auf die inneren Organe und auf einen großen Teil der Gefäße des Körperblutkreislaufes, so daß auch dieser unterstützt wird.

Bei der Unterstützung des Lungenblutkreislaufes und insbesondere bei der Ansteuerung des hierfür vorgesehenen Unterdruckerzeugers an der Atemmaske werden dieselben Vorrichtungen, wie z.B. Überwachungsgeräte und Steuergeräte eingesetzt, die auch schon bei der flüssigkeitsgefüllten Kammer zur Unterstützung des Körperblutkrieslaufes beschrieben wurden.

Wenn der systolische Blutdruck in Richtung des diastolischen Blutdruckes abnimmt, verringert sich auch der in der Atemmaske impulsartig aufgebaute Unterdruck, was zu einer erneuten Verengung der Blutgefäße führt. Dementsprechend sinkt das Gefäßvolumen und das Blut wird mit ausreichender Sauerstoffmenge aus den Gasaustauschgefäßen in den linken Vorhof des Herzens gefördert. Dies ist eine Voraussetzung für die vollständige Füllung der linken Herzkammer und hilft damit, den linken Herzkreislauf, d.h. den Körperblutkreislauf besser zu durchbluten.

Nach dem vollständigen Blutkreislauf-Unterstützungsvorgang wird die Luftzufuhr durch erneute Ansteuerung der entsprechenden Ventile wieder geöffnet, so daß der Patient ungehindert weiteratmen kann. Der gesamte Unterstützungsvorgang,d.h. die Unterbrechung der Luftzufuhr, Erzeugung eines impulsartigen Unterdruckes und erneute Öffnung der Luftzufuhr, dauert ebenso wie bei der Kammer nur etwa 0,1 Sek. Hierbei müssen die unterstützend einwirkenden Unterdruckimpulse exakt herzschlagsynchron, bzw. mit dem Herzschlag korreliert sein, um eine Unterstützung des Blutkreislaufes und Entlastung des Herzens zu bewirken. Nicht synchrone Unterdruckimpulse wirken sich hingegen nachteilig und gesundheitsschädlich aus.

In einer weiteren Ausgestaltung der Erfindung ist es möglich, beide Vorrichtungen, d.h. die flüssigkeitsgefüllte Kammer und die erfindungsgemäße Atemmaske miteinander zu kombinieren.

Ein zu behandelnder Patient liegt dementsprechend in einer flüssigkeitsgefüllten Kammer ein und wird nunmehr nicht über eine normale Atemmaske sondern über die erfindungsgemäße Atemmaske mit Atemluft versorgt. Dementsprechend können beide Blutkreisläufe, d.h. der Körper- und der Lungenblutkreislauf gleichzeitig unterstützt werden, wodurch sich eine maximale Wirkung ergibt.

Diese Wirkung kann zusätzlich auf eine bestimmte Teilfläche des Körpers bzw. auf ein bestimmtes inneres Organ konzentriert werden. Die Wirkung auf einen bestimmten Bereich des Körpers, z. B. ein bestimmtes Organ, wird hier maßgeblich von der Liegeposition des Patienten, und somit von der Wirkung des hydrostatischen Blutdruckes sowie von der Position der flüssigkeitsgefüllten Kammer und der Stärke und Form der auf den Körper bzw. das Atmungssystem ausgeübten Unterdruckimpulse bestimmt.

Eine maximale Wirkung erfolgt dementsprechend an der Stelle, wo die Unterdruckimpulse zusammentreffen und der hydrostatische Blutdruck am größten ist.

Ausführungsbeispiele der Erfindung sind in den nachfolgenden Abbildungen dargestellt.

Es zeigen:
- Figur 1: Eine flüssigkeitsgefüllte Kammer zur Unterstützung des Körperblutkreislaufes
- Figur 2: Eine Atemmaske zur Unterstützung des Lungenblutkreislaufes
- Figur 3: eine alternative Ausbildung der Atemmaske
- Figur 4: eine Kombination von Kammer und Atemmaske zur gleichzeitigen Unterstützung sowohl des Körper- als auch des Lungenblutkreislaufes

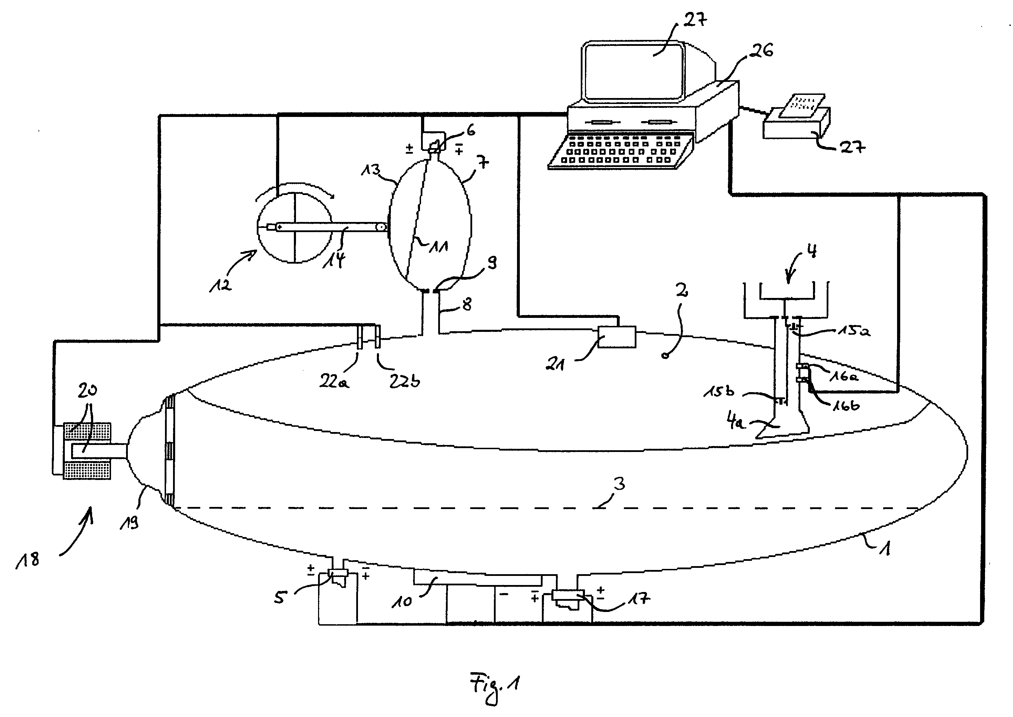

In der Fig. 1 ist eine Vorrichtung zur Unterstützung des Körperblutkreislaufes und zur Entlastung des Herzens dargestellt. Diese Vorrichtung umfaßt eine mit Flüssigkeit füllbare Kammer 1, die durch einen Deckel 2 verschlossen ist.

Durch die, mittels des Deckels 2 verschlossene Öffnung kann ein zu behandelnder Patient in die Kammer einsteigen und sich auf dem Liegenetz 3 hinlegen.

Um zu gewährleisten, daß der zu behandelnde Patient bei mit Flüssigkeit gefüllter Kammer mit Atemluft versorgt werden kann, wird der Patient an eine entsprechende Vorrichtung 4 angeschlossen, die im wesentlichen aus einer Atemmaske 4a besteht. Diese Atemmaske deckt sowohl Mund als auch Nase des Patienten ab und ermöglicht so die Luftversorgung.

Nach dem Verschließen des Deckels 2 wird die Kammer 1 mit Flüssigkeit gefüllt, wozu an der Kammer ein insbesondere elektronisch betätigtes Einlaßventil 5 vorgesehen ist. Während der Befüllung mit der Flüssigkeit, die vorteilhafter vortemperiert ist, kann die aus der Kammer verdrängte Luft über ein Entlüftungsventil 6 entweichen, welches an einem Kompensationsbehälter 7 angeordnet ist.

Dieser Kompensationsbehälter 7 ist über eine Verbindungsleitung 8 mit der Kammer 1 verbunden, wobei die Verbindungsleitung 8 bevorzugt über ein ansteuerbares und insbesondere elektromagnetisches Ventil 9 verschließbar ist. Alternativ kann statt des Ventils auch eine Widerstandsdüse in der Verbindungsleitung vorgesehene sein. Nach voll-ständiger Befüllung der Kammer 1 und des Kompensations-behälters 7 werden sowohl das Einlaßventil 5 als auch das Entlüftungsventil 6 geschlossen. Dieses geschieht durch entsprechende Ansteuerung der beispielsweise elektro-magnetisch betätigten Ventile mittels eines Steuergeräts, welches durch einen Computer 26 gegeben sein kann.

Um dem in der Kammer einliegenden Patienten die Behandlung so angenehm wie möglich zu machen, ist die Kammer 1, beziehungsweise wenigstens der Deckel 2 aus einem durchsichtigen Material hergestellt. Somit kann in gewissem Umfang eine Kommunikation zwischen dem Patienten und dem behandelnden Arzt gewährleistet werden. Weiterhin sind während der Behandlung die Gehörgänge des Patienten zu verschließen,

um negative Auswirkung des Unterdruckes, insbesondere auf das Trommelfell zu verhindern.

Zusätzlich ist an der Kammer eine ebenfalls über den Computer 26 steuerbare Heizvorrichtung 10 vorgesehen, mit der die Flüssigkeit in der Kammer derart temperiert werden kann, daß sie von dem Patienten als angenehm empfunden wird.

Da sich während der Atmung das Körpervolumen des Patienten verändert, d.h. mit der Atemfrequenz zu bzw. wieder abnimmt, muß gewährleistet sein, daß z.B. bei der Einatmung eine der Volumenzunahme des Körpers entsprechende Flüssigkeitsmenge aus der Kammer verdrängt werden kann, um den Druck in der Kammer durch die Atembewegung nicht zu verändern.

Hierzu ist der an der Kammer 1 angeordnete Kompensationsbehälter 7 vorgesehen.

Gegenüber dem Atmosphärendruck ist das Volumen des Kompensationsbehälters 7 wenigstens an einer Seite mit einer Membran 11 abgeschlossen. Diese Membran 11 besteht aus einem elastischen verformbaren Material, so daß diese Membran unter dem Druck des bei der Atmung in den Kompensationsbehälter 7 verdrängten Wassers verformt wird.

Aufgrund der einfachen Verformbarkeit der Membran 11 wird sich hierdurch im Gesamtsystem der Druck nicht verändern. Zur aktiven Beatmung des Patienten ist an dem Kompensationsbehälter 7 eine Antriebsvorrichtung 12 vorgesehen, mit der die Membran 11 direkt oder indirekt über eine weitere Membran 13 verformbar ist. Dies Antriebsvorrichtung ist wiederum über den Computer 26 steuerbar.

Zwischen den Membranen 11 und 13, kann hierbei ein abgeschlossenes Luftvolumen ausgebildet sein, um eine sanftere Wirkung zu erzielen. Dementsprechend wird bei dem in der Figur 1 dargestellten Antrieb 12 mittels der Schubstange 14 das Membranpaar 11/13 bewegt, wodurch aktiv entweder Flüssigkeit aus dem Kompensationsbehälter 7 in die Kammer gedrückt bzw. aus der Kammer 1 in den Kompensationsbehälter 7 eingesaugt wird. Stange 14 und Antrieb 12 sind hierbei an das Atmungsvolumen des Patienten anzupassen.

Durch diesen Vorgang wird der auf den Körper des Patienten einwirkende Druck in der Kammer geändert, so daß auf Grund der Inkompressibilität der Flüssigkeit der Körper des Patienten aktiv ausgedehnt bzw. komprimiert wird.

Dementsprechend wird das Lungenvolumen des Patienten geändert, so daß mit Hilfe der Antriebsvorrichtung 12 ein Luftausstoß aus der Lunge bzw. ein Einsaugen von Luft in die Lunge bewirkt wird. Der Patient kann somit über die Vorrichtung 12 aktiv beatmet werden.

Die assistierte Beatmung des in der Kammer liegenden Patienten mittels der Vorrichtung 12, die die Membranen 11 und 13 des Kompensationsbehälters 7 antreibt, hat die Vorteile, daß bei dieser Art der Beatmung, die Lunge nicht mit Preßluft aufgeblasen wird, sondern sich ein künstlich hervorgerufener Atemmechanismus ergibt, welcher der natürlichen Atmung des Menschen entspricht.

Da die Lunge und die Blutgefäße der Lunge durch diese Methode keinerlei Druck ausgesetzt werden, zeigt diese Beatmungsmethode keinerlei Nebenwirkungen.

Um eine Pendelatmung des immer gleichen Luftvolumens in der Beatmungsvorrichtung 4 zu verhindern, weist diese die Strömungsrichtung der Luft bestimmende Ventile 15a und 15b auf. Diese können gleichzeitig als Widerstandsdüsen ausgebildet sein, um eine passive Lungenblutkreislaufunterstützung während der Behandlung zu erreichen. Weiterhin sind an der Atemvorrichtung 4 Drucksensoren 16a und 16b vorgesehen, mit denen untere und obere Druckgrenzwerte der Atemluft sowie der Momentandruck in der Maske messtechnisch erfaßbar sind. Auch diese Meßwerte können zur weiteren Auswertung und Steuerung des Gesamtsystems dem Steuergerät bzw. Computer 26 zugeführt werden.

Im Notfall, wenn z.B. der Strom an der Vorrichtung ausfällt, bzw. der Patient in Panik gerät, wird automatisch oder durch den Patienten hervorgerufen, ein elektrisch ansteuerbares Ablaßventil 17 geöffnet, durch welches die Flüssigkeit aus der Kammer 1 entweichen kann. Gleichzeitig mit der Öffnung des Ablaßventiles 17 wird ebenfalls das Ent-/Belüftungsventil 6 geöffnet um für die entweichende Flüssigkeit Luft in die Kammer einströmen zu lassen.

Für die Körperblutkreislauf-Unterstützung des in der flüssigkeitsgefüllten Kammer 1 einliegenden Patienten ist z.B. am Ende der Kammer ein Unterdruckerzeuger 18 angeordnet. Dieser Unterdruckerzeuger 18 besteht im wesentlichen aus einer elastisch verformbaren Membran 19, die beispielsweise mittels eines elektromagnetischen Antriebes 20 verformt werden kann.

Die Membran 19 schließt zumindest an einer definierten Stelle die Kammer 1 gegenüber der Atmosphäre ab. Hierdurch ist gewährleistet, daß durch eine Verformung der Membran 19 mittels des Antriebes 20 das Volumen in der Kammer 1 geändert werden kann.

Mit Hilfe des Unterdruckerzeugers 18 kann somit auf den, in der Flüssigkeitskammer 1 liegenden Patienten bei entsprechender Ansteuerung des Antriebes 20 impulsartig und herzschlagsynchron aktiv ein Unterdruck ausgeübt werden. Während der Körper aktiv herzschlagsynchronen Unterdruckimpulsen ausgesetzt wird, unterstützt die Atemmaske 4 den Lungenblutkreislauf nur passiv mit dem Unterdruckimpuls, der zwischen dem Atmungssystem des Patienten und Widerstandsdüsen 15 in der Atemmaske entsteht.

Um eine entsprechende Ansteuerung des Antriebes 20 zu ermöglichen, sind in der Kammer nicht dargestellte Geräte vorgesehen, mit denen die Herz- bzw. Blutkreislauftätigkeit des Patienten überwacht werden kann.

Im einfachsten Fall können mit diesen Geräten die Herzströme oder Herztöne aufgenommen werden. Um die Überwachungsdaten, wie z.B. EKG-Ströme einem außerhalb der Kammer 1 angeordneten Steuergerät 26 zuzuführen, weist die Kammer 1 bzw. der Deckel 2 der Kammer einen Anschluß 21 auf, durch den die entsprechenden Signale von den Überwachungsgeräten zum Steuergerät 26 leitbar sind. Die Überwachungs- und Ansteuerungsdaten können weiterhin mittls einer Ausgabeeinheit 27, z.B. einem Monitor oder eines Druckers angezeigt und/oder protokolliert werden.

Innerhalb des Steuergerätes wird anhand von Patientendaten sowie weiterer für die Vorrichtung spezifischer Daten, die von dem behandelnden Arzt eingegeben werden und anderer Überwachungsdaten, wie z.B. dem Druck in der Kammer, ein z.B. elektrischer oder elektronischer Impuls abgeleitet, der synchron bzw. korreliert zum Herzschlag erzeugt wird.

Dieser Impuls dient zur Ansteuerung des Antriebes 20, so daß ebenso herzschlagsynchron, bzw. korreliert zum Herzschlag der Unterdruck in der Kammer erzeugt werden kann. Diese impulsartige Erzeugung des Unterdruckes, die beispielsweise nur über die Dauer von 0,1 Sek. erfolgt, führt zu den eingangs genannten Vorteilen und Wirkungen, nämlich einer impulsartigen Erweiterung der Blutgefäße und somit zu der genannten Körperblutkreislauf-Unterstützung und Herzentlastung.

Um zu verhindern, daß während der Unterdruckerzeugung, Flüssigkeit aus dem Kompensationsbehälter 7 in die Kammer strömt und somit den Unterdruck-Effekt kompensiert, wird für den Zeitraum der impulsartigen Unterdruck-Erzeugung durch einen weiteren Ansteuerungsimpuls des Steuergerätes, das elektronisch betätigbare Ventil 9 in der Verbindungsleitung 8 des Kompensationsbehälters geschlossen.

Alternativ zu dem elektromagentisch betätigbaren Ventil 9 am Kompensationsbehälter 7 kann an dieser Stelle auch eine Widerstandsdüse vorgesehen sein, die der aufgrund des kurzen Impulses schnell strömenden Flüssigkeit einen derart hohen Widerstand entgegenbringt, daß eine Kompensation des Druckes bei Ausüben des herzschlagsynchronen, impulsartigen Unterdruckes vermieden wird. Eine Kompensation des Druckes in der Kammer bei der hingegen relativ langsam vorgehenden Atmung des Patienten, bleibt dennoch, auch durch die Widerstandsdüsen gewährleistet.

Um zu gewährleisten, daß sich die in der Kammer erzeugten Drücke innerhalb eines gesundheitlich unbedenklichen Rahmens bewegen, sind entsprechende Grenzwertbedingungen durch den behandelnden Arzt oder werkseitig in dem Steuergerät 26 programmiert und können durch ebenfalls mit dem Steuergerät verbundene Drucksensoren 22a und 22b überwacht werden. Hierbei kann beispielsweise der eine Drucksensor einem unteren und der andere einem oberen Druckgrenzwert zugeordnet sein. Es ist ebenso möglich mit diesen Drucksensoren den momentanen in der Kammer herrschenden Druck zu erfassen und mittels des Steuergerätes 26 das Signal zu Ansteuerung des Antriebs 20 abzuleiten.

Neben den technischen Vorrichtungen zeigt die Kammer 1 weiterhin das konstruktive Merkmal einer im wesentlichen runden und beinah eiförmigen Form.

Hierdurch ergibt sich bei der Kammer ein vergrößerter Resonanzwiderstand, so daß verhindert werden kann, daß die Kammer durch die herzschlagsynchron und impulsartig ausgeübten Unterdruckimpulse in Eigenschwingung gerät.

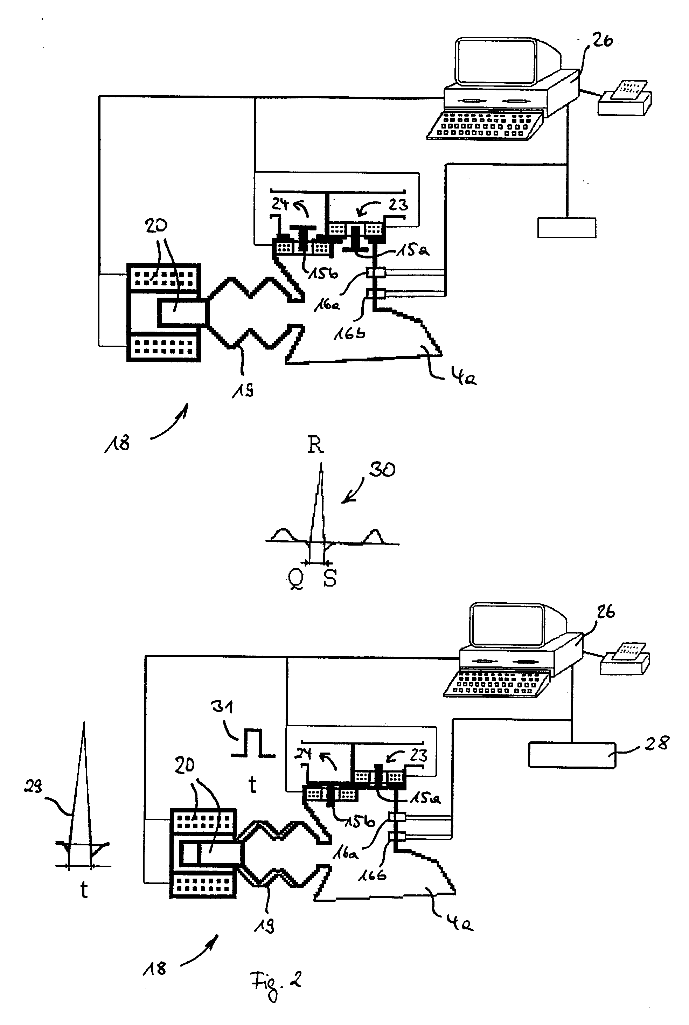

In der Figur 2 ist eine Vorrichtung zur Unterstützung des Lungenblutkreislaufes und zur Entlastung des Herzens dargestellt, die im wesentlichen aus einer Atemmaske 4a besteht, über die ein Patient mit Atemluft versorgt wird. Beim Atmen strömt die Atemluft durch einen Kanal 23 und durch das richtungsebende Ventil 15a in die Maske ein.

Die Luft wird vom Patienten über die Maske 4a durch Mund oder Nase ein und anschließend wieder ausgeatmet. Hierbei entweicht die ausgeatmete Luft über das ebenfalls richtungsgebende Ventil 15b durch den Kanal 24.

Um in dem Atemvolumen, welches aus dem Volumen der Maske bzw. der gesamten Vorrichtung und dem Lungenvolumen des Patienten gebildet wird, einen impulsartigen und herzschlagsynchronen Unterdruckimpuls zu erzeugen, weist diese Vorrichtung genauso wie die vorangehend diskutierte Kammer einen Unterdruckerzeuger 18 auf, der im wesentlichen aus einer elastischen verformbaren Membran 19 und einem elektromagnetischen Antrieb 20 besteht.

Durch Ansteuerung des elektromagnetischen Antriebes 20 kann entsprechend der Verformung der Membran 19 impulsartig das Atemvolumen geändert werden, so daß ein Unterdruck im Atemtrakt des Patienten erzeugbar ist. Die Ansteuerung des Antriebes 20 erfolgt hierbei in äquivalenter Weise durch ein Steuergerät 26, wie es schon bei der Kammer der Figur 1 beschrieben wurde.

In dieses Steuergerät werden Überwachungsdaten beispielsweise von einem EKG oder einem anderen Gerät 28 eingespeist, so daß aus diesen Daten und eventuell weiterer vorgegebener Daten der Ansteuerungsimpuls 29 für den Antrieb 20 ableitbar ist. Dieser Ansteuerungssimpuls wird beispielsweise aus den üblichen QRS-Strömen 30 des Herzens, wie sie ebenfalls in der Figur 2 mittig dargestellt sind, abgeleitet.

Der Ansteuerungsimpuls 29 bewirkt eine Bewegung der Membran, wie es in der Figur 2 unten dargestellt ist, so daß sich das Volumen in der Atemmaske ändert, insbesondere vergrößert, um den Unterdruck zu erzeugen.

Um zu vermeiden, daß beispielsweise bei der Vergrößerung des Atemmaskenvolumens Luft aus den Kanälen 23 bzw. 24 nachströmt und somit den erzeugten Unterdruck kompensiert, werden die richtungsbestimmenden Ventile 15a bzw. 15b durch einen weiteren Steuerimpuls 31 elektrisch angesteuert und geschlossen. Statt der Ventile 15a,b können auch Widerstandsdüsen vorgesehen sein, die eine Druckkompensation verhindern. Dieser Steuerimpuls 31 der ebenfalls von dem Steuergerät zur Verfügung gestellt werden kann, ist z.B. nur unwesentlich länger als der Steuerimpuls, der den Antrieb 20 ansteuert.

Die Dauer dieser Steuerimpulse beträgt hierbei etwa nur 0,1 Sek., wobei darauf zu achten ist, daß die erzeugten Impulse exakt herzschlagsynchron bzw. mit dem Herzschlag korreliert sind.

Durch den im Atmungstrakt des Patienten impulsartig und herzschlagsynchron erzeugten Unterdruck werden die eingangs genannten therapeutischen Wirkungen erzielt. Dementsprechend kommt es zu einem relativen Unterdruck in den Blutgefäßen der Lunge und ebenfalls durch die Ausbreitung des Unterdrucks über das Zwerchfell, bei den weiteren Körperblutgefäßen.

Insofern ist, wie vorangehend erklärt, eine Unterstützung des Blutkreislaufes und eine Entlastung des Herzens möglich.

Zur Überwachung der in der Atemmaske vorherrschenden Drücke weist diese 2 Drucksensoren 16a und 16b auf, mit denen sowohl ein oberer als auch ein unterer Grenzwertdruck überwachbar ist. Die von diesen Drucksensoren ermittelten Meßwerte werden ebenfalls dem Steuergerät 26 zugeleitet, welches aus dem insgesamt zur Verfügung gestellten Daten die Ansteuerungsimpulse 29/31 sowohl für den Antrieb 20 als auch für die Ventile 15 ableitet.

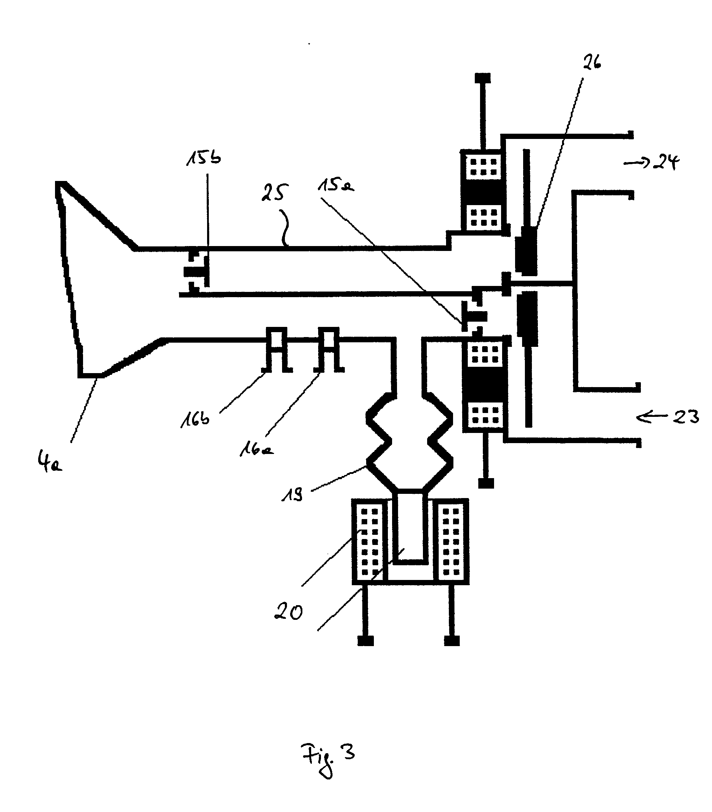

Die Figur 3 zeigt eine weitere Ausführungsform nach Figur 2, bei dem im wesentlichen nur das Maskenteil 4a über eine längere Verbindung 25 mit den Luftkanälen 23 und 24 verbunden ist.

Weiterhin ist die Funktion der die Luftrichtung bestimmenden Ventile 15a und 15b von der Funktion der abriegelnden Ventile 26 getrennt. Ansonsten weist diese Vorrichtung dieselben technischen Merkmale auf, wie die Atemvorrichtung nach Figur 2.

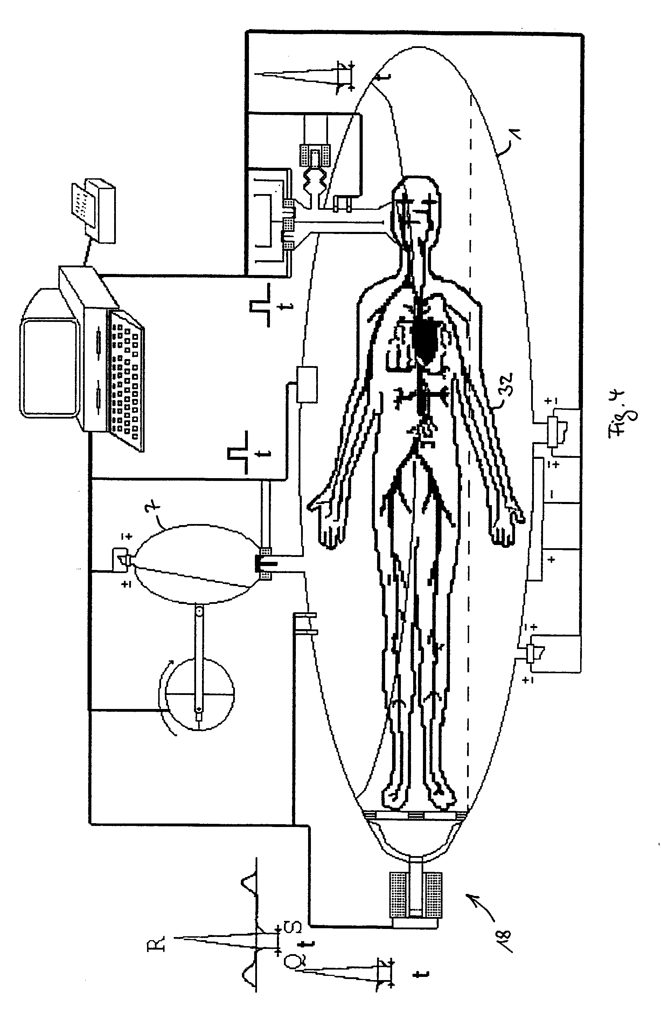

Die Atemvorrichtung nach Figur 3 hat jedoch gegenüber der Atemvorrichtung nach Figur 2 den Vorteil, daß sie mit der Vorrichtung zur Körperblutkreislauf-Unterstützung gemäß Figur 1 kombinierbar ist. Eine derartige Kombination ist in der Figur 4 dargestellt.

Diese Figur 4 zeigt im wesentlichen, die aus der Figur 1 bekannte flüssigkeitsgefüllte Kammer 1, in der ein zu behandelnder Patient 32 einliegt. Dieser Patient ist hier nur aus darstellerischen Gründen in Aufsicht abgebildet.

Neben den bereits aus der Figur 1 bekannten technischen Vorrichtungen, wie beispielsweise Unterdruckerzeuger 18 und Kompensationsbehälter 7 zeigt die Figur 4 als Beatmungsmaske, die erfindungsgemäße Vorrichtung zur LungenblutkreislaufUnterstützung entsprechend der Figur 3. Somit ist es möglich, während der Behandlung sowohl gezielt den Körperblutkreislauf als auch den Lungenblutkreislauf gleichzeitig zu unterstützen.

Wie bereits eingangs erwähnt kann dementsprechend nach Figur 4 gezielt ein inneres Organ behandelt werden, da die kresilaufunterstützenden Wirkungen dort am größten sind, wo die erzeugten Drücke örtlich gleichzeitig zur Wirkung kommen. Dies kann insbesondere mittels des hydrostatischen Druckes vorherbestimmt werden, so daß gezielt einzelne Organe behandelbar sind.

Um hier ebenfalls die Auswirkungen des hydrostatischen Druckes mitzuberücksichtigen kann die Position des Patienten 32 in der Kammer und ebenfalls die Lage der Kammer selbst geändert werden. Beispielsweise ist die Kammer aus der horizontalen Position in die vertikale Position verschwenkbar.

Es ist ergänzend zu erwähnen, daß die bei der Kammer und der Atemmaske beschriebenen Unterdruck-Erzeuger einen zueinander äquivalenten Aufbau haben und sämtliche bei den jeweiligen Vorrichtungen erwähnten Merkmale auf beide Unterdruck-Erzeuger gleichsam zutreffen. Dies betrifft insbesondere die Steuerung mittels des Steuergeräts 26 anhand der zur Verfügung gestellten Daten/Meßwerte.

Die Vorrichtung nach Figur 4 vereint sämtliche durch die einzelnen Vorrichtungen möglichen Wirkungen und ermöglicht sogar darüber hinausgehende Behandlungen. Insbesondere ist es möglich, die genannten erfindungsgemäßen Vorrichtungen für prophylaktische Therapien einzusetzen.

Es kann somit Krankheiten und unnatürlichen Alterungsprozzessen vorgebeugt werden, das Streßrisiko vor chirugischen Eingriffen kann gesenkt und nach diesem Eingriff die Erholungsphase deutlich verkürzt werden. Viele Krankheiten, die besonders auf mangelnde Durchblutung und Stoffwechselstörungen zurückzuführen sind, sind mittels der erfindungsgemäßen Vorrichtungen behandelbar bzw. heilbar.

Aufgrund der örtlich vorherbestimmbaren Wirkungsfelder können spezielle Medikamenten-Heilungsmethoden, wie z.B. die Chemotherapie gezielter eingesetzt und somit optimiert werden.

Auch zur Akuttherapie, wie beispielsweise bei Gehirnschlag, Herzschwäche oder Herzinfarkt ist eine Behandlung mit den erfindungsgemäßen Vorrichtungen zu empfehlen, da sich diese allgemein stabilisierend und entlastend auf den Blutkreislauf auswirken. Insbesondere bei erhöhtem Gehirndruck macht sich eine Unterdrucktherapie mit den erfindungsgemäßen Vorrichtungen vorteilhaft bemerkbar.

## Patentansprüche

1. Vorrichtung zur Unterstützung des Körperblutkreislaufes und zur Entlastung des Herzens mittels auf den Körper eines Patienten ausübbaren Unterdrucks mit einer Kammer in der ein zu behandelnder Patient einliegt und mit einer Atemvorrichtung zur Atemluftversorgung des Patienten **dadurch gekennzeichnet, daß** die Kammer mit Flüssigkeit füllbar ist und in ihr impulsartig und herzschlagsynchron auf den Körper des Patienten einwirkender Unterdruck erzeugbar ist.

2. Vorrichtung nach Anspruch 1, **dadurch gekennzeichnet, daß** an der Kammer ein Unterdruck-Erzeuger angeordnet ist, mit dem impulsartig das Kammervolumen änderbar ist.

3. Vorrichtung nach einem der vorherigen Ansprüche, **dadurch gekennzeichnet, daß** mit der Kammer ein Kompensationsbehälter mit variablem Inhaltsvolumen verbunden ist.

4. Vorrichtung nach einem der vorherigen Ansprüche, **dadurch gekennzeichnet, daß** die Verbindung zwischen Kammer und Kompensationsbehälter über ein ansteuerbares Ventil verschließbar ist und/oder eine Widerstandsdüse aufweist.

5. Vorrichtung nach einem der vorherigen Ansprüche, **dadurch gekennzeichnet, daß** das Inhaltsvolumen des Kompensationsbehälters durch eine elastische verformbare Membran begrenzt ist.

6. Vorrichtung nach einem der vorherigen Ansprüche, **dadurch gekennzeichnet, daß** die Membran aktiv bewegbar ist.

7. Vorrichtung nach einem der vorherigen Ansprüche, **dadurch gekennzeichnet, daß** die Kammer ein Liegenetz aufweist.

8. Vorrichtung nach einem der vorherigen Ansprüche, **dadurch gekennzeichnet, daß** die Flüssigkeit in der Kammer eine derart hohe Dichte aufweist, daß der Patient in der Flüssigkeit schwebt.

9. Vorrichtung nach einem der vorherigen Ansprüche, **dadurch gekennzeichnet, daß** an der Kammer eine Heizvorrichtung zur Temperierung der Kammerflüssigkeit vorgesehen ist.

10. Vorrichtung nach einem der vorherigen Ansprüche, **dadurch gekennzeichnet, daß** an der Kammer ansteuerbare Zufluß-, Abfluß- und Ent- bzw. Belüftungsventile angeordnet sind.

11. Vorrichtung nach einem der vorherigen Ansprüche, **dadurch gekennzeichnet, daß** die Kammer aus der horizontalen Position verschwenkbar ist.

12. Vorrichtung zur Unterstützung des Lungenblutkreislaufes und zur Entlastung des Herzens mit einer Atemmaske, über die der Patient mit Atemluft versorgt wird, **dadurch gekennzeichnet, daß** im durch Atemmaske und Patient gebildeten Atemvolumen impulsartig und herzschlagsynchron Unterdruck erzeugbar ist.

13. Vorrichtung nach Anspruch 12, **dadurch gekennzeichnet, daß** das Atemvolumen durch das Luftvolumen in der Atemmaske und das Lungenvolumen des Patienten gegeben ist.

14. Vorrichtung nach einem der vorherigen Ansprüche, **dadurch gekennzeichnet, daß** an der Atemmaske ein Unterdruck-Erzeuger angeordnet ist, mit dem impulsartig das Atemvolumen änderbar ist.

15. Vorrichtung nach einem der vorherigen Ansprüche, **dadurch gekennzeichnet, daß** in der Atemmaske die Strömungsrichtung der Atemluft bestimmende Ventile angeordnet sind.

16. Vorrichtung nach einem der vorherigen Ansprüche, **dadurch gekennzeichnet, daß** in der Atemmaske ansteuerbare Ventile und/oder Widerstandsdüsen angeordnet sind.

17. Vorrichtung zur Kreislaufunterstützung und zur Entlastung des Herzens **dadurch gekennzeichnet, daß** eine Vorrichtung zur Körperblutkreislaufunterstützung und eine Vorrichtung zur Lungenblutkreislaufunterstützung nach den vorherigen Ansprüchen miteinander kombiniert sind.

18. Vorrichtung nach einem der vorherigen Ansprüche, **dadurch gekennzeichnet, daß** der Unterdruck-Erzeuger eine elastische verformbare Membran umfaßt, die insbesondere von einem elektromagnetischen Antrieb bewegbar ist.

19. Vorrichtung nach einem der vorherigen Ansprüche, **dadurch gekennzeichnet, daß** sie Geräte zur Überwachung der Herz- und/oder Blutkreislauftätigkeit umfaßt.

20. Vorrichtung nach einem der vorherigen Ansprüche, **dadurch gekennzeichnet, daß** mit den Geräten Herzströme (Aktionsströme), Herztöne oder Ultraschallbilder des Herzens, die Herztätigkeit, der Blutdruck oder der Blutstrom überwachbar sind.

21. Vorrichtung nach einem der vorherigen Ansprüche, **dadurch gekennzeichnet, daß** mittels eines an den Geräten angeschlossenen Steuergeräts Impulse zur Ansteuerung der Unterdruck-Erzeuger erzeugbar sind.

22. Vorrichtung nach einem der vorherigen Ansprüche, **dadurch gekennzeichnet, daß** die Impulse in ihrer Form und/oder Höhe einstellbar sind.

23. Vorrichtung nach einem der vorherigen Ansprüche, **dadurch gekennzeichnet, daß** an ihr Drucksensoren vorgesehen sind, insbesondere zur Überwachung eines unteren und eines oberen in der Vorrichtung herrschenden Druckgrenzwertes.
